# EUROPEAN PATENT SPECIFICATION

(11) **EP 4 023 810 B1**
(45) Date of publication and mention of the grant of the patent: **28.01.2026**
(21) Application number: 20859606.4
(22) Date of filing: 19.08.2020
(51) Int. Cl.: D04B 21/18, G01N 5/00, G01N 33/36

(54) **METHOD FOR EVALUATING FIBER PRODUCTS**
VERFAHREN ZUR BEWERTUNG VON FASERPRODUKTEN
PROCÉDÉ D'ÉVALUATION D'ARTICLES EN FIBRES

(30) Priority: 30.08.2019 JP 2019158068
(43) Date of publication of application: 06.07.2022
(73) Proprietor: Toray Industries, Inc., Tokyo 103-8666 (JP)
(72) Inventor: KIMURA, Chika, Otsu-shi, Shiga 520-2141 (JP); TOYABE, Keigo, Otsu-shi, Shiga 520-2141 (JP); IGAKURA, Saori, Otsu-shi, Shiga 520-2141 (JP); SUZUKI, Hidetoshi, Otsu-shi, Shiga 520-2141 (JP)
(74) Representative: Mewburn Ellis LLP
(86) International application number: PCT/JP2020/031220
(87) International publication number: WO 2021/039520

(56) References cited:
- WO-A1-2017/173215
- WO-A1-2018/048329
- CN-A- 109 569 086
- CN-U- 206 285 589
- JP-A- 2019 505 351
- NAPPER IMOGEN E ET AL: "Release of synthetic microplastic plastic fibres from domestic washing machines: Effects of fabric type and washing conditions", MARINE POLLUTION BULLETIN, OXFORD, GB, vol. 112, no. 1, 26 September 2016 (2016-09-26), pages 39 - 45, XP029763570, ISSN: 0025-326X, DOI: 10.1016/J.MARPOLBUL.2016.09.025
- BOGATY HERMAN ET AL: "MEASUREMENT OF RATE OF FLOW OF WATER THROUGH FILTER PAPER Research Paper RP163", PART OF JOURNAL OF RESEARCH OF THE NATIONAL BUREAU OF STANDARDS VOLUME 33, 1 November 1944 (1944-11-01), pages 1 - 10, XP093068197, Retrieved from the Internet <URL:https://nvlpubs.nist.gov/nistpubs/jres/33/jresv33n5p353_a1b.pdf> [retrieved on 20230727]
- DE FALCO FRANCESCA ET AL: "The contribution of washing processes of synthetic clothes to microplastic pollution", vol. 9, no. 1, 30 April 2019 (2019-04-30), XP055793381, Retrieved from the Internet <URL:http://www.nature.com/articles/s41598-019-43023-x> DOI: 10.1038/s41598-019-43023-x

## Description

### TECHNICAL FIELD

The present invention relates to a method for evaluating a fiber product.

### BACKGROUND ART

Recently, there is a concern about an adverse effect on the ecosystem due to plastic wastes in oceans or rivers being taken into organisms. In particular, there is a concern that a plastic container is microminiaturized by ultraviolet rays or the like to become micro-sized plastic fragments, but a waste reduction and a relevance to a micro-plastic problem are also discussed for all plastic products.

Synthetic fibers are often used in a fiber product currently on the market, in particular, a functional fiber product manufactured for sports and outdoor applications, and the synthetic fibers may fall out as laundry fragments from a cut portion or the like during washing. For example, a bulky mid-layer having a heat-retaining property represented by a fleece is known, and this is generally produced by subjecting a surface of a fabric to a raising process and fluffing fibers. This material may cause fiber fragments to fall out from a raised portion as well as the cut portion during washing, and tends to generate more fiber fragments than a non-raised material.

So far, JIS L1096 (2010), JIS L0844, ISO 7768 (2009), and the like have been standardized as evaluation methods for washing. All these methods are test methods focusing on the quality of an object to be washed such as dimensional stability, washing fastness, wrinkle characteristics, or the like of the object to be washed. In addition, since a fiber product is exposed to high mechanical stress such as washing to cause damage and the like, a standard test for detecting the mechanical stress has also been proposed (for example, Patent Document 1). On the other hand, recently, considering fiber fragments generated during washing as a problem, a washing bag having an enhanced ability to filter fiber fragments has been proposed in order to filter the fiber fragments (for example, Patent Document 2). Non-patent Documents 1 and 2 each disclose a study examining release of fibres during washing.

### PRIOR ART DOCUMENTS

### PATENT DOCUMENTS

Patent Document 1: JP 2006-521475
Patent Document 2: JP 2019-505351

### NON-PATENT DOCUMENTS

Non-patent Document 1: I.E. Napper et al., Marine Pollution Bulletin (2016), 112, 39-45
Non-patent Document 2: F. De Falco et al., Scientific Reports (2019), 9

### SUMMARY OF THE INVENTION

### PROBLEMS TO BE SOLVED BY THE INVENTION

In general, the fiber fragments generated during washing or the like are removed from washing liquid, washing wastewater, or the like and then discarded. Considering the possibility of various problems such as an increase in waste, a wastewater treatment load, and a maintenance load of a washing machine, it is more preferable as the amount of fiber fragments is smaller. These problems can be solved by a fiber product that suppresses the amount of fiber fragments, but a method for evaluating a fiber product that suppresses the amount of fiber fragments generated during washing has not been proposed so far. In addition, although there is a difference in performance between a product having a large amount of fiber fragments and a product having a small amount of fiber fragments, there is no means for comparing these products, which is problematic.

For example, the JIS or ISO standard described above is an evaluation method focusing on an object to be washed during washing, and does not describe fiber fragments generated from the object to be washed at all. In addition, also in Patent Document 1, an evaluation target is a device, and there is no description of a method for evaluating a fiber product or fiber fragments. On the other hand, Patent Document 2 is focused on fiber fragments and describes that a fiber product is put in a washing bag and washed, and fiber fragments generated from the fiber product are not flown into wastewater. However, there is no description of a method for evaluating fiber fragments generated from a fiber product. This is because it is premised on generation of fiber fragments from a fiber product, and there is no point of view to provide a method for quantitatively evaluating fiber fragments generated from a fiber product during washing and thus to create a fiber product that suppresses generation of fiber fragments.

Therefore, a new issue has arisen in creating a method for quantitatively evaluating fiber fragments generated from a fiber product with excellent reproducibility.

An object of the present invention is to provide a method for evaluating fiber fragments generated from a fiber product during washing.

### SOLUTIONS TO THE PROBLEMS

In order to solve the above problem, the present invention has the following configuration. A method for evaluating a fiber product in accordance with claim 1, including: washing at least one fiber product by a washing machine and washing conditions defined in ISO 6330 (2012); filtering fiber fragments discharged from a wastewater outlet of the washing machine by a filter with a sieve opening of 5 to 20 µm; and measuring a weight of the fiber fragments, in which a water permeability of the filter is 300 seconds or shorter when measured in the following order,
water permeability measurement procedure:
(a) a sheet cut into a circular shape having a diameter of 20 cm from the filter is folded into four and set in a triangular funnel having a diameter of 22 cm;
(b) 300 ml of distilled water is poured and a time until a last one drop falls from the funnel after an initial one drop falls from the funnel is measured; and
(c) in a case where a next drop does not fall for longer than 5 minutes, the last one drop is determined as a last one drop.

In some embodiments of the method for evaluating a fiber product, the filter is attached to a drain hose of the washing machine.

In some embodiments of the method for evaluating a fiber product, the sieve opening is 9 µm or more and 15 µm or less.

In some embodiments of the method for evaluating a fiber product, a filter with a sieve opening of 150 to 5,000 µm is further provided between the wastewater outlet of the washing machine and the filter.

In some embodiments of the method for evaluating a fiber product, the washing machine is cleaned by performing each of rinse and drain at least once before the washing.

In some embodiments of the method for evaluating a fiber product, a detergent is not put in the washing.

In some embodiments of the method for evaluating a fiber product, one fiber product is used in the washing.

In some embodiments of the method for evaluating a fiber product, the washing machine is a C-type standard washing machine.

In some embodiments of the method for evaluating a fiber product, the washing condition is a 4N method.

### EFFECTS OF THE INVENTION

According to the present invention, a method for evaluating a fiber product to quantitatively evaluate the amount of fiber fragments generated with excellent reproducibility can be provided. Therefore, a fiber product that suppresses fiber fragments is easily created.

### BRIEF DESCRIPTION OF THE DRAWINGS

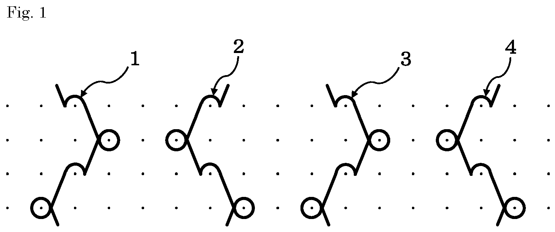

Fig. 1 is a view of a knitted pattern of each of a non-elastic fiber and an elastic fiber in Production Example 1.

### EMBODIMENTS OF THE INVENTION

A fiber product to be evaluated in the present invention is not particularly limited, and it is preferable that at least a part of a fabric included in the fiber product contains synthetic fibers. The fabric used in the present invention may be a woven fabric, a knitted fabric, or a non-woven fabric. In a case where the fabric is a woven fabric, a weave pattern is not particularly limited, and examples thereof include plain weave, twill weave, satin weave, modified plain weave, modified twill weave, modified satin weave, fancy weave, single weave, double pattern, multiple pattern, warp pile weave, weft pile weave, and interlaced weave. In addition, the fabric is a knitted fabric, a knitted pattern is not particularly limited, and examples thereof include circular knitted fabric, weft knitted fabric, warp knitted fabric (including tricot fabric and russell fabric), pile fabric, plain stitch, plain knit, rib-knit, smooth knit (double knit), rib stitch, purl knit, denbigh pattern, cord pattern, atlas pattern, chain pattern, and insertion pattern.

Examples of the synthetic fiber constituting the fabric used for the fiber product include a filament yarn and a spun yarn. The spun yarn may contain natural fibers as long as it at least partially contains various synthetic fibers. The filament yarn includes a drawn yarn and various twisted yarns. The type of twisted yarn is not particularly limited, and examples thereof include a false twisted yarn, a false twisted-fused yarn, and a medium twisted yarn.

A material of the synthetic fiber is not particularly limited. Examples thereof include polymers such as polyethylene terephthalate, polyethylene naphthalate, polybutylene terephthalate, polytrimethylene terephthalate, polypropylene, polyolefin, polycarbonate, polyacrylate, polyamide, polylactic acid, polyurethane, and polyphenylene sulfide, and copolymers thereof. These materials may contain various additives such as an inorganic substance such as titanium oxide, silica, or barium oxide, a colorant such as carbon black, a dye, or a pigment, a flame retardant, a fluorescent brightener, an antioxidant, and an ultraviolet absorber.

A cross-sectional shape of the fiber is not particularly limited, and may be various cross-sectional shapes such as a flat cross section, a polygonal cross section such as a triangle, a quadrangle, a hexagon, or an octagon, a dumbbell-shaped cross section partially having concave and convex portions, a Y-shaped cross section, and a star-shaped cross section, in addition to a perfect circular cross section.

A method for dyeing the fabric is not particularly limited. Examples thereof include scouring, relaxation, heat setting, dyeing processing, weight reduction processing, and functional processing. In the functional processing, water repellency, antistatic processing, flame retardancy, moisture absorption, antistatic processing, antibacterial processing, flexible finishing, and other known functional processing can be performed, if necessary. In a case where the synthetic fiber contained in the fabric includes polyurethane fibers, heat setting is preferably performed at 190°C or higher. When the temperature is equal to or higher than this temperature, the polyurethane fibers are fused, and it is possible to suppress fraying of the fibers and generation of the fiber fragments that fall off.

A method for cutting the fabric is not particularly limited, and it is preferable to select ultrasonic cutting. In the cutting method, a cut portion is welded, such that it is possible to suppress fraying of the fibers and generation of the fiber fragments that fall off.

A method for sewing the fiber product is not particularly limited, and a piping treatment or an embedding treatment using a sealing tape is preferable. By applying these treatments, it is possible to prevent the cut portion of the fabric from being exposed during washing and to suppress fraying of the fibers and generation of the fiber fragments that fall off.

In addition, the fiber product after sewing is preferably subjected to a post-treatment of any of washing, air blowing, and air suction. In a case where these treatments are performed, the fiber fragments attached to the cut portion of the fabric and the fabric surface can fall off in advance, and the amount of fiber fragments generated during washing can be suppressed.

One aspect of a method for evaluating a fiber product of the present invention is a method for performing a washing test of a fiber product, filtering fiber fragments using a filter attached to a drain hose or the like of a washing machine, for example, a filtering bag (filter), and evaluating a weight of the fiber fragments. Evaluation accuracy can be improved by evaluating the weight of the generated fiber fragments instead of evaluating a change in weight of the fiber product that is an object to be washed. A specific method is as follows.

It is preferable that before the evaluation, washing is performed according to ISO 6330 (2012) without putting an object to be washed or a detergent in the washing machine and the washing machine is cleaned. It is possible to prevent the influence of the residue of the fiber fragments and the like due to the washing performed in advance and to improve the reproducibility of the evaluation result. Conventional JIS or ISO for evaluating an object to be washed does not require the method described above, and thus, the above description is not defined therein. However, it is a preferable characteristic step in the evaluation method of the present invention for evaluating fiber fragments. It is more preferable to clean the washing machine by performing rinsing and dehydrating steps once or more each without putting an object to be washed and a detergent. In addition, it is preferable that conditions are the same as the washing conditions for evaluation or the amount of water and washing machine power are set to the maximum.

In the evaluation method of the present invention, a washing machine defined in ISO 6330 (2012) is used. There are various different types and shapes of washing machines, and the washing machines have different physical actions (washing machine power) applied to an object to be washed during washing. Among various washing machines used in general households, the washing machine defined in ISO 6330 (2012) has a basic function and thus is used for evaluation of a fiber product in the present invention. An A-type standard washing machine, a B-type standard washing machine, and a C-type standard washing machine are defined in ISO 6330 (2012), and any one of these washing machines can be used in the present invention. Among them, an A-type standard washing machine or a C-type standard washing machine that is actually used at home in many countries can be preferably used in the evaluation method of the present invention. Further, it is possible to more preferably use a C-type standard washing machine capable of more accurately measuring the fiber fragments by suppressing reattachment of the fallen fiber fragments to the object to be washed or the like due to a large amount of water used during washing.

The washing in the present invention is performed under conditions defined in ISO 6330 (2012). Several types of washing conditions are defined for each of the washing machines described above, and any of the washing conditions can be adopted in the present invention. For example, 13 types of washing methods and 7 types of washing methods are defined for an A-type standard washing machine and a C-type standard washing machine that are preferably used in the present invention, respectively. Among them, as the washing conditions in the present invention, a 4N method, 4M method, 3N method, or 3M method for the A-type standard washing machine and a 4N method, 4M method, 3N method, or 3M method for the C-type standard washing machine are preferable, and the 4N method for the C-type standard washing machine is more preferable, in terms of high reproducibility of the amount of fiber fragments generated from a fiber product and suitability for evaluating the amount of fiber fragments. In general, in a case where the washing machine power is too weak due to a washing machine or washing conditions, a proportion of a relatively large incidentally attached yarn or the like in the filtered fiber fragments increases, and accurate evaluation tends to be difficult. On the contrary, in a case where the washing machine power is too strong, fragments other than the fiber fragments generated by washing are generated due to damage of the fiber product, and accurate evaluation also tends to be difficult. By the washing machine and washing conditions of the present invention, in particular, the preferred washing machine and washing conditions of the present invention described above, potential fiber fragments that may be generated during washing of a fiber product are efficiently generated, and it is easy to filter and evaluate the fiber fragments by a filter with a sieve opening defined in the present invention.

The fiber fragments discharged from a wastewater outlet of the washing machine are filtered by attaching a filter (hereinafter, may be referred to as a "first filter") with a sieve opening of 5 to 20 µm to a drain hose or the like of the washing machine. According to the washing machine and washing conditions adopted in the evaluation method of the present invention, a weight of the fiber fragments can be evaluated with excellent reproducibility by setting the sieve opening within the range of the present invention. When the sieve opening is less than 5 µm, a drainage speed is lowered and the fiber fragments are likely to remain in the washing machine, and it is difficult to collect and evaluate the generated fiber fragments with excellent reproducibility. In addition, it is required to set the sieve opening to 20 µm or less in order to filter the fiber fragments. From the viewpoint of a balance, the sieve opening is preferably set to 9 µm or more. In addition, the sieve opening is preferably set to 15 µm or less, and is more preferably set to 12 µm or less.

In the evaluation method of the present invention, it is preferable that in addition to the first filter, a filter with a sieve opening of 150 to 5,000 µm (hereinafter, may be referred to as a "second filter") is further provided between the wastewater outlet of the washing machine and the first filter. The fiber fragments filtered by the first filter can be used to evaluate fine fiber fragments that fall off from the fiber product. However, in a case where large fiber fragments that are not preferable as an evaluation target are generated, the large fiber fragments affect the evaluation result. On the other hand, the evaluation using the first filter of the present invention becomes preferably quantitative with excellent reproducibility by removing the influence of large fiber fragments by the second filter. It is preferable that a weight of the fiber fragments filtered by the second filter is similarly measured as reference information and the measured weight is added to the evaluation result. Note that in the present invention, the sieve opening can be obtained by measuring 10 points with a microscope of 500 magnifications and rounding off the average value to the first decimal place. In a case where a hole of the sieve opening has a round shape, a maximum diameter is measured. In a case of a quadrangular shape, the longer one of a warp and a weft is measured. Note that in a case where a filter is incorporated into the washing machine, the incorporated filter is removed because it affects the filtering amount.

In addition, the filter used in the present invention is not limited to a sheet shape, a bag shape, a box shape, or the like, and may have any structure as long as there is the sieve opening as described above and water permeates. Specific examples thereof include a metal mesh, a perforated metal sheet, a perforated filter, a non-woven fabric, a woven fabric, and a knitted fabric, and a woven fabric is more preferably used because it is excellent in versatility, water permeability, and filtering performance and has high reproducibility.

A material of the filter may be either a metal or plastic.

Plastic is not particularly limited. Examples thereof include polymers such as polyethylene terephthalate, polyethylene naphthalate, polybutylene terephthalate, polytrimethylene terephthalate, polypropylene, polyolefin, polycarbonate, polyacrylate, polyamide, polylactic acid, polyurethane, and polyphenylene sulfide, and copolymers thereof.

The water permeability of the filter used in the present invention is 300 seconds or shorter. When the water permeability is 300 seconds or shorter, a decrease in drainage speed from the washing machine is suppressed, and an amount of fiber fragments remaining in the washing machine is small, so that the fiber fragments can be collected with excellent reproducibility. The water permeability is more preferably 200 seconds or shorter, and still more preferably 100 seconds or shorter. In addition, a lower limit thereof is not particularly limited, and is substantially 5 seconds or longer. The water permeability can be appropriately adjusted by a porosity, water absorbency, and the like of the filter. In a case where the filter has a fiber structure, the water permeability can be adjusted by also a density, fineness, and pattern.

Note that in the present invention, the water permeability is measured by the following evaluation method.
(a) A sheet cut into a circular shape having a diameter of 20 cm from the filter is folded into four and set in a triangular funnel having a diameter of 22 cm. That is, the sheet is folded in half twice, and one to be into a bag shape is opened in a conical shape and set in a triangular funnel. This is a method similar to a four-fold method of a filter paper although there are different portions such as a folding portion and a torn portion.
(b) 300 ml of distilled water is poured and a time until a last one drop falls from the funnel after an initial one drop falls from the funnel is measured.
(c) In a case where a next drop does not fall for longer than 5 minutes, the last one drop is determined as a last one drop.

Such a filter is not special, and may be appropriately selected from commercially available sheets and the like. A specific example thereof includes a filtering bag produced using a "nylon screen" NY10-HC (manufactured by FLON INDUSTRY).

In the method for evaluating a fiber product of the present invention, at least one fiber product to be evaluated is put in a washing machine in a state where a filter is attached, and the fiber product is washed by the washing machine and washing conditions described above. However, a detergent and a loading fabric are not used. After the washing, a weight of fiber fragments attached to a filter, for example, the "nylon screen" is measured. One fiber product refers to one fiber product regardless of a shape, size, and weight. The amount of fiber fragments generated does not necessarily depend on the size and weight, and definition of the number of fiber products is more excellent in reproducibility or quantitativeness. In the present invention, in principle, it is preferable to perform relative comparison in the same or similar product group. In addition, it is more preferable to append a weight, shape, and size of the fiber product, and a fiber fragment ratio obtained by the amount of fiber fragments in the weight of the fiber product to the evaluation result in comparison with another fiber product. In addition, in order to improve reproducibility or quantitativeness, it is preferable to prepare a fiber product to be a reference and similarly wash and compare the fiber product. In addition, the number of fiber products is at least one, and it is also allowable to wash a plurality of fiber products at the same time. On the other hand, in order to prepare 2 kg of a fiber product as described in ISO 6330 (2012), in a case of 100 g of one piece of inner wear, it is required to prepare 20 pieces. In consideration of this, the number of fiber products is preferably one from the viewpoint of convenience of evaluation.

A method for measuring the weight of the fiber fragments collected by the filter is not particularly limited to a method in which a filter is dried to measure a weight, a method in which fiber fragments are washed off from a filter using pure water and then the fiber fragments are collected through filtration by a filter with a sieve opening of 0.1 to 20 µm, and the like. A method for measuring a weight of fiber fragments by drying a filter is preferable because it is simple and hardly causes an operation error. In addition, a method of collecting fiber fragments by filtration is preferable because there is no occurrence of an error caused by a change in weight of the fiber fragments due to moisture absorption and desorption of the filter, outflow of a material of the filter, and the like. In this case, in order to collect the fibers filtered by the filter, the sieve opening of the filter is set to be smaller than the sieve opening of the filter. A specific example of the method of collecting fiber fragments by filtration is described below. After absolute drying, suction filtration is performed using a filter whose weight is measured in advance, for example, a polycarbonate membrane (K040A047A, manufactured by Advantec Toyo Kaisha, Ltd.). The filter and the fiber fragments after filtration are dried at 105°C for 1 hour, the weight is measured, and a difference from the weight before filtration is taken as the amount of fiber fragments. As for conditions of the absolute drying and weight measurement, heating is performed at 105°C for 1 hour, and then, the weight is measured after the temperature and humidity are controlled to 20°C and 65% RH, respectively. The amount of fiber fragments filtered in the present evaluation is preferably 10.0 (mg/one fiber product) or less, and more preferably 6.0 (mg/one fiber product) or less as a target fiber product.

Note that a method in which one fiber product is put in a washing bag and washed, and fiber fragments attached to the washing bag are filtered and evaluated (hereinafter, may be referred to as a "washing bag method") is also considered. In comparison with the evaluation method of the present invention, the method for measuring the amount of fiber fragments in the filtering method is as follows. A "nylon screen" NY10-HC (manufactured by FLON INDUSTRY) is used to prepare an isosceles triangular washing bag having one side of 1 m. A fiber product to be evaluated is put in the washing bag, and washing is performed according to an ISO 6330C 4N method. However, a detergent and a loading fabric are not used. After the washing, the fiber product is removed, and suction filtration is performed using a polycarbonate membrane (K040A047A, manufactured by Advantec Toyo Kaisha, Ltd.) in which a weight of the fiber fragments attached to the washing bag is measured in advance. The polycarbonate membrane and the fiber fragments after filtration are dried at 105°C for 1 hour, the weight is measured, and a difference from the weight before filtration is taken as the amount of fiber fragments.

Even in this washing bag method, the fiber fragments can be evaluated. However, when the fiber product is put in the washing bag, washing machine power is weakened, and an amount of fiber fragments generated is smaller than that in actual washing. Further, since the fiber fragments are re-attached to the fiber product, reproducibility of the amount of fiber fragments is poor, and it is difficult to relatively compare the amount of fiber fragments generated from the fiber product.

Since the reproducibility is deteriorated due to the influence of a detergent on the amount of fiber fragments generated, washing is preferably performed in an amount equal to or less than the amount defined in JIS or ISO, and washing is more preferably performed without putting a detergent. In a case where a detergent is put, it is required to consider insoluble components of the detergent. In addition, since fiber fragments are also generated from a loading fabric, it is preferable not to use the loading fabric.

### [Examples]

Hereinafter, the method for evaluating a fiber product of the present invention will be specifically described with reference to Examples.

### (1) Water Permeability

(a) A sheet cut into a circular shape having a diameter of 20 cm from a filter was folded into four and set in a triangular funnel having a diameter of 22 cm.
(b) 300 ml of distilled water was poured and a time until a last one drop fell from the funnel after an initial one drop fell from the funnel was measured.
(c) In a case where a next drop did not fall for longer than 5 minutes, the last one drop was determined as a last one drop.

### (2) Sieve Opening

A surface of the filter was imaged at a magnification of 500 times using a digital microscope VHX-7000 (manufactured by Keyence Corporation), and 10 opening portions were measured. An average value of the obtained values was rounded off to the first decimal place and taken as a value of a sieve opening. In a case where the hole had a round shape, a maximum diameter was measured, and in a case where the hole had a square shape, the longer diameter was measured.

### (3) Measurement of Weight of Fiber Fragments

Suction filtration was performed on an aqueous solution containing fiber fragments using a polycarbonate membrane (K040A047A, hole diameter of 0.4 µm, manufactured by Advantec Toyo Kaisha, Ltd.) whose weight was measured in advance. The polycarbonate membrane and the fiber fragments after filtration were dried at 105°C for 1 hour, the weight was measured, and a difference from the weight before filtration was taken as the amount of fiber fragments.

### [Production Example 1 of Fiber Product]

A warp knitted fabric composed of knitted loops formed of non-elastic fibers obtained using nylon fibers (40 dtex-34F) and elastic fibers obtained using urethane fibers (22 dtex) was produced. The warp knitted fabric is knitted with a knitted pattern illustrated in Fig. 1. That is, the warp knitted fabric has a non-elastic fiber knitted pattern formed of a pair of half-set knits of first non-elastic fibers 1 and second non-elastic fibers 2 threaded in one-in and one-out half sets through a first reed and a second reed, respectively, and an elastic fiber knitted pattern formed of a pair of half-set knits of first elastic fibers 3 and second elastic fibers 4 threaded in one-in and one-out half sets through a third reed and a fourth reed, respectively. In addition, as illustrated in Fig. 1, in the non-elastic fiber knitted pattern, the first non-elastic fiber 1 is composed of repeating units of 10/12/23/21//, and the second non-elastic fiber 2 is composed of repeating units of 23/21/10/12//. In the non-elastic fiber knitted pattern, four courses are repeated units, and each repeating unit is deflected left and right within a range of two wales. As illustrated in Fig. 1, the elastic fiber knitted pattern formed of the first elastic fibers 3 and the second elastic fibers 4 is also the same knitted pattern as the non-elastic fiber knitted pattern. As for a mixing ratio of the materials, nylon is 84% and polyurethane is 16%. The obtained fabric was subjected to a heat treatment at 190°C for 1 minute. Another processing was performed according to dyeing processing conditions of a common warp knitted fabric to obtain a knitted fabric. A common T-shirt having a V-shaped collar was produced using the obtained knitted fabric. In the production of the T-shirt, a collar, a hem, and a cuff were not sewed, and only common cutting was finished. The sewing of an armpit portion, a front and back body joint portion, and a cuff and body joint portion was performed by a common treatment method for the T-shirt.

### [Production Example 2 of Fiber Product]

A single circular knitting machine was used to produce a circular knitted fabric with a plain knitted pattern using polyester fibers (66 dtex-96F) as non-elastic fibers and polyurethane fibers (22 dtex) as elastic fibers. As for a mixing ratio of the materials, polyester is 90% and polyurethane is 10%. Processing was performed on the obtained fabric according to dyeing processing conditions of a common circular knitted fabric to obtain a knitted fabric. A common T-shirt having a U-shaped collar was produced using the obtained knitted fabric. In the production of the T-shirt, unlike Production Example 1, all a collar, a hem, and a cuff, an armpit portion, a front and back body joint portion, and a cuff and a body joint portion were sewed by a common method for a T-shirt.

### [Production Example 3 of Fiber Product]

A 1:1 interknitted fabric with a plain knitted pattern was produced using acryl/rayon spun yarns (metric number: 1/64) for knitted loops 1 formed of non-elastic fibers, and using polyester fibers (84 dtex-72F) and polyurethane elastic fibers (33 dtex) for knitted loops 2 formed of non-elastic fibers and elastic fibers. As for a mixing ratio of the materials, acryl is 28%, rayon is 34%, polyester is 33%, and polyurethane is 5%. Processing was performed on the obtained fabric according to dyeing processing conditions of a common circular knitted fabric to obtain a knitted fabric. A common nine-tenth length T-shirt having a U-shaped collar was produced using the obtained knitted fabric, and the T-shirt was sewed in the same manner as that of Production Example 2.

### [Examples 1 to 3]

The fiber product obtained in each of Production Examples 1 to 3 and a C-type standard washing machine described in ISO 6330 (2012) were used. First, for cleaning of a washing machine, 7 kg of AQW-V 700E (manufactured by AQUA Co., Ltd.) was used by a C4N method of ISO 6330 (2012), and rinse and drain were performed twice without putting an object to be washed. Specifically, a course was carefully set, the amount of water was set to 40 L, a washing time was set to 15 minutes, rinse was set to two times, dehydration was set to 7 minutes, a washing water temperature was set to 40°C, and a rinsing water temperature was set to room temperature. Next, a filtering bag produced using a "nylon screen" NY10-HC (manufactured by FLON INDUSTRY) with a sieve opening of 11.3 µm and a water permeability of 82 seconds was attached to a drain hose of the washing machine. Thereafter, one fiber product to be evaluated was put in the washing machine, and washing was performed under washing conditions of a C4N method of ISO 6330. However, a detergent and a loading fabric were not used. After the washing, suction filtration of the fiber fragments attached to the "nylon screen" was performed using a polycarbonate membrane (K040A047A, manufactured by Advantec Toyo Kaisha, Ltd.) of which a weight was measured in advance. The polycarbonate membrane and the fiber fragments after filtration were dried at 105°C for 1 hour, the weight was measured, and a difference from the weight before filtration was taken as the amount of fiber fragments generated.

The amount of fiber fragments filtered by a wastewater filtering method was 9.8 mg/one fiber product (weight of fiber fragments/weight of fiber product = 114 ppm) in Example 1 using the fiber product of Production Example 1, 34.3 mg/one fiber product (279 ppm) in Example 2 using the fiber product of Production Example 2, and 72.0 mg/one fiber product (444 ppm) in Example 3 using the fiber product of Production Example 3.

In Example 1, since the polyurethane fibers were fused by the high temperature heat treatment at 190°C, it was found that the generation of the fiber fragments from the cut portion was suppressed and the amount of fiber fragments generated measured after the washing was smaller than in each of Examples 2 and 3 in which the polyurethane fibers were not fused. Since the knitted fabric formed of filaments was used in Example 2 and the knitted fabric formed of staples and filaments was used in Example 3, the results were shown that the fiber fragments were likely to be generated from the staples.

### [Example 4]

The same operation was performed in the same manner as that of Example 1, except that the fiber product obtained by Production Example 1 was used, and a stainless steel mesh (number of meshes: 4.7, manufactured by Clever Co., Ltd.) with a sieve opening of 4,004 µm was attached between the wastewater outlet of the washing machine and the filtering bag. The obtained fiber fragments were almost equivalent to 9.6 mg/one fiber product (111 ppm), but it was found that a standard deviation was small when a plurality of other fiber products obtained by Production Example 1 were evaluated.

### [Comparative Examples 1 to 3]

The same operation (washing bag method) was performed in the same manner as that of Example 1, except that a washing bag was used instead of attaching the filter to the drain hose. The amount of fiber fragments filtered by the washing bag method was 3.9 mg/one fiber product (45 ppm) in Comparative Example 1 using the fiber product of Production Example 1, 11.0 mg/one fiber product (89 ppm) in Comparative Example 2 using the fiber product of Production Example 2, and 10.1 mg/one fiber product (62 ppm) in Comparative Example 3 using the fiber product of Production Example 3. In Comparative Example 2, more fiber fragments were generated than in Comparative Example 3, and the results were shown that the tendency was different from those obtained in Example 2 and Example 3.

In addition, the amount of fiber fragments obtained by the washing bag method is generally smaller than that obtained by the wastewater filtering method using no washing bag, but it is because the amount of fiber fragments generated is reduced by a reduction in wafer flow applied to the fiber product by the washing bag and limitation of the flow of the fiber product during the washing test.

### [Comparative Examples 4 to 6]

The same operation was performed in the same manner as those of Examples 1 to 3, except that a "nylon screen" NY50-HC (manufactured by FLON INDUSTRY) with a sieve opening of 50 µm was used. The amount of fiber fragments filtered was 4.0 mg/one fiber product (45 ppm) in Comparative Example 4 using the fiber product of Production Example 1, 11.5 mg/one fiber product (93 ppm) in Comparative Example 5 using the fiber product of Production Example 2, and 21.7 mg/one fiber product in Comparative Example 6 using the fiber product of Production Example 3. It was estimated that the results were shown that the amount of fiber fragments was smaller than that in each of Examples and a small amount of the fiber fragments flowed.

### [Reference Example]

The same operation was performed in the same manner as that of Example 1, except that an object to be washed was not put and the cleaning of the washing machine was not performed. A calculated weight of the fiber fragments was 4.6 mg. It was estimated that fibers with various colors were not mixed with the fiber fragments and the fiber product that had been washed in the past was included. The results were shown that the cleaning of the washing machine was important for evaluating the fiber fragments by washing.

### DESCRIPTION OF REFERENCE SIGNS

- 1:: First non-elastic fiber
- 2:: Second non-elastic fiber
- 3:: First elastic fiber
- 4:: Second elastic fiber

## Claims

1. A method for evaluating a fiber product, comprising:
washing at least one fiber product by a washing machine and washing conditions defined in ISO 6330 (2012);
filtering fiber fragments discharged from a wastewater outlet of the washing machine by a filter with a sieve opening of 5 to 20 µm; and
measuring a weight of the fiber fragments, **characterized in that** a water permeability of the filter is 300 seconds or shorter when measured in the following order,
water permeability measurement procedure:
(a) a sheet cut into a circular shape having a diameter of 20 cm from the filter is folded into four and set in a triangular funnel having a diameter of 22 cm;
(b) 300 ml of distilled water is poured and a time until a last one drop falls from the funnel after an initial one drop falls from the funnel is measured; and
(c) in a case where a next drop does not fall for longer than 5 minutes, the last one drop is determined as a last one drop.

2. The method for evaluating a fiber product according to claim 1, wherein the filter is attached to a drain hose of the washing machine.

3. The method for evaluating a fiber product according to claim 1 or 2, wherein the sieve opening is 9 µm or more and 15 µm or less.

4. The method for evaluating a fiber product according to any one of claims 1 to 3, wherein a second filter with a sieve opening of 150 to 5,000 µm is further provided between the wastewater outlet of the washing machine and the filter.

5. The method for evaluating a fiber product according to any one of claims 1 to 4, wherein the washing machine is cleaned by performing each of rinse and drain at least once before the washing.

6. The method for evaluating a fiber product according to any one of claims 1 to 5, wherein a detergent is not put in the washing.

7. The method for evaluating a fiber product according to any one of claims 1 to 6, wherein one fiber product is used in the washing.

8. The method for evaluating a fiber product according to any one of claims 1 to 7, wherein the washing machine is a C-type standard washing machine.

9. The method for evaluating a fiber product according to any one of claims 1 to 8, wherein the washing condition is a 4N method.

## Patentansprüche

1. Verfahren zum Bewerten eines Faserprodukts, umfassend:
Waschen von zumindest einem Faserprodukt durch eine Waschmaschine und Waschbedingungen gemäß der Definition von ISO 6330 (2012);
Abfiltrieren von Faserfragmenten, die über einen Abwasserauslass der Waschmaschine abgeleitet wurden, durch ein Filter mit einer Sieböffnung von 5 bis 20 µm; und
Messen eines Gewichts der Faserfragmente,
**dadurch gekennzeichnet, dass** eine Wasserdurchlässigkeit des Filters 300 Sekunden oder kürzer ist, wenn sie in der folgenden Abfolge gemessen wird,
Wasserdurchlässigkeitsmessverfahren:
(a) ein aus dem Filter kreisförmig ausgeschnittenes Blatt mit einem Durchmesser von 20 cm wird in vier Teile gefaltet und in einen dreieckigen Trichter mit einem Durchmesser von 22 cm gegeben;
(b) es werden 300 ml destilliertes Wasser eingefüllt und ein Zeitraum, bis ein letzter Tropfen aus dem Trichter fällt, nachdem ein erster Tropfen aus dem Trichter gefallen ist, gemessen; und
(c) in dem Fall, dass mehr als 5 Minuten lang kein weiterer Tropfen fällt, wird bestimmt, dass der letzte Tropfen ein letzter Tropfen ist.

2. Verfahren zum Bewerten eines Faserprodukts nach Anspruch 1, wobei das Filter an einem Abflussschlauch der Waschmaschine befestigt ist.

3. Verfahren zum Bewerten eines Faserprodukts nach Anspruch 1 oder 2, wobei die Sieböffnung 9 µm oder mehr und 15 µm oder weniger beträgt.

4. Verfahren zum Bewerten eines Faserprodukts nach einem der Ansprüche 1 bis 3, wobei ferner ein zweites Filter mit einer Sieböffnung von 150 bis 5.000 µm zwischen dem Abwasserauslass der Waschmaschine und dem Filter bereitgestellt ist.

5. Verfahren zum Bewerten eines Faserprodukts nach einem der Ansprüche 1 bis 4, wobei die Waschmaschine vor dem Waschen durch zumindest einmaliges Durchführen von jedem aus einem Spül- und einem Auslassvorgang gereinigt wird.

6. Verfahren zum Bewerten eines Faserprodukts nach einem der Ansprüche 1 bis 5, wobei bei dem Waschvorgang kein Waschmittel verwendet wird.

7. Verfahren zum Bewerten eines Faserprodukts nach einem der Ansprüche 1 bis 6, wobei ein Faserprodukt bei dem Waschvorgang verwendet wird.

8. Verfahren zum Bewerten eines Faserprodukts nach einem der Ansprüche 1 bis 7, wobei die Waschmaschine eine C-Typ-Standard-Waschmaschine ist.

9. Verfahren zum Bewerten eines Faserprodukts nach einem der Ansprüche 1 bis 8, wobei die Waschbedingung ein 4N-Verfahren ist.

## Revendications

1. Procédé d'évaluation d'un produit en fibres, comprenant les étapes consistant à :
laver au moins un produit en fibres par l'intermédiaire d'une machine à laver et de conditions de lavage définies dans la norme ISO 6330 (2012) ;
filtrer des fragments de fibres évacués à partir d'une sortie d'eaux usées de la machine à laver à l'aide d'un filtre dont l'ouverture de tamis est de 5 à 20 µm ; et
mesurer un poids des fragments de fibres, **caractérisé en ce que** la perméabilité à l'eau du filtre est de 300 secondes ou moins lorsqu'elle est mesurée dans l'ordre suivant,
Procédure de mesure de perméabilité à l'eau :
(a) une feuille découpée en une forme circulaire présentant un diamètre de 20 cm à partir du filtre est pliée en quatre et placée dans un entonnoir triangulaire présentant un diamètre de 22 cm ;
(b) 300 ml d'eau distillée sont versés, et le temps nécessaire pour qu'une dernière goutte tombe de l'entonnoir après qu'une première goutte soit tombée de l'entonnoir et mesuré ;
(c) dans le cas où une goutte suivante ne tombe pas pendant plus de 5 minutes, la dernière goutte est considérée comme la dernière goutte.

2. Procédé d'évaluation d'un produit en fibres selon la revendication 1, dans lequel le filtre est fixé à un tuyau de vidange de la machine à laver.

3. Procédé d'évaluation d'un produit en fibres selon la revendication 1 ou 2, dans lequel l'ouverture de tamis est de 9 µm ou plus et de 15 µm ou moins.

4. Procédé d'évaluation d'un produit en fibres selon l'une quelconque des revendications 1 à 3, dans lequel un second filtre avec une ouverture de tamis de 150 à 5 000 µm est en outre prévu entre la sortie d'eaux usées de la machine à laver et le filtre.

5. Procédé d'évaluation d'un produit en fibres selon l'une quelconque des revendications 1 à 4, dans lequel la machine à laver est nettoyée en effectuant un rinçage et une vidange au moins une fois avant le lavage.

6. Procédé d'évaluation d'un produit en fibres selon l'une quelconque des revendications 1 à 5, dans lequel aucun détergent n'est ajouté au lavage.

7. Procédé d'évaluation d'un produit en fibres selon l'une quelconque des revendications 1 à 6, dans lequel un seul produit en fibres est utilisé dans le lavage.

8. Procédé d'évaluation d'un produit en fibres selon l'une quelconque des revendications 1 à 7, dans lequel la machine à laver est une machine à laver standard de type C.

9. Procédé d'évaluation d'un produit en fibres selon l'une quelconque des revendications 1 à 8, dans lequel la condition de lavage est une méthode 4N.
